(19) **Europäisches Patentamt**

**European Patent Office**

**Office européen des brevets**

(11) **EP 1 612 225 A1**

(12) **EUROPEAN PATENT APPLICATION**
published in accordance with Art. 158(3) EPC

(43) Date of publication:
**04.01.2006 Bulletin 2006/01**

(51) Int Cl.:
*C08B 37/00* (1974.07)   *A61K 31/736* (2000.01)
*A61P 1/14* (2000.01)   *A61P 35/00* (2000.01)
*A61P 3/06* (2000.01)   *A61P 3/10* (2000.01)
*A23L 1/308* (1985.01)

(21) Application number: **04726810.7**

(22) Date of filing: **09.04.2004**

(86) International application number:
**PCT/JP2004/005146**

(87) International publication number:
**WO 2004/089992 (21.10.2004 Gazette 2004/43)**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **09.04.2003 JP 2003105903**

(71) Applicants:
• **Phillips Hydrocolloids Research Limited**
  **London W1S 4AQ (GB)**
• **SAN-EI GEN F.F.I., INC.**
  **Toyonaka-shi,**
  **Osaka 561-8588 (JP)**

(72) Inventors:
• **AL-ASSAF, Saphwan**
  **Wrexham, LL11 2BW (GB)**
• **PHILLIPS, G.O.,**
  **Phillips Hydrocolloids Res. Ltd.**
  **London W1S 4AQ (GB)**
• **SASAKI, Yasushi,**
  **c/o SAN-EI GEN F.F.I., INC.**
  **Toyonaka-shi, Osaka561-8588 (JP)**
• **KATAYAMA, Tsuyoshi**
  **Toyonaka-shi, Osaka561-8588 (JP)**

(74) Representative: **Müller-Boré & Partner**
**Patentanwälte**
**Grafinger Strasse 2**
**81671 München (DE)**

(54) **MODIFIED ACACIA AND USE THEREOF**

(57)    The present invention aims to provide modified gum arabic having an increased total dietary fiber content and use thereof. The modified gum arabic of the present invention, which is obtained by heating gum arabic, has a total dietary fiber content (determined by AOAC method) of not less than 90%, and preferably has a weight-average molecular weight of not less than 1,000,000. This modified gum Arabic is usable as a dietary fiber material or an additive for enriching dietary fiber to be used in foods, drinks and medicines. Therefore, the present invention provides foods, drinks or medicines containing the above-described modified gum Arabic as dietary fiber material or an additive for enriching dietary fiber.

EP 1 612 225 A1

**Description**

TECHNICAL FIELD

The present invention relates to modified gum arabic. In particular, the invention relates to gum arabic modified so as to contain a high total dietary fiber content.

BACKGROUND OF THE INVENTION

Gum arabic is a natural resin prepared by drying the rubbery exudate from the trunks and branches of plants that belong to the genus Acacia of the Leguminasae family, in particular, *Acacia senegal* and *Acacia seyal.* Gum arabic is highly soluble in water and its aqueous solution provides high emulsion stability, protective colloidability, and filmforming ability even at low concentrations, and therefore has been widely used as an emulsifier, thickener, stabilizer, and coating agent ("Industrial Gums, Polysaccharides and their Derivatives", second edition, Academic Press, New York and London, 1973, pp 197-263). Furthermore, gum arabic contains dietary fiber that is not digested by human digestive enzymes. The content of dietary fiber in gum arabic measured by enzymatic-HPLC methods is 80 to 90% (Monthly Food Chemical, 2002-6, pp 85-89), 85% measured by the AOAC method, and not less than 70% measured by the Englyst method (Monthly Food Chemical, 1997-7, pp 102-104).

In recent years, many researchers have indicated that an insufficient dietary fiber intake caused by a westernized Japanese diet is relevant to the increase in patients suffering from diabetes, obesity, arteriosclerosis and the like lifestyle-induced diseases. Therefore, the physiological functions of dietary fiber are attracting attention, and the necessity of intaking dietary fiber is highlighted.

As described above, because gum arabic has a significantly lower viscosity than macromolecular polysaccharides, which are a different kind of dietary fiber, gum arabic can be used at high concentrations and has a high level of safety. Gum arabic can be added to foods, including drinks, without adversely affecting their texture, and therefore gum arabic is a useful material for intaking a large amount of dietary fiber. Accordingly, there is a demand for developing foods that contain gum arabic as dietary fiber.

DISCLOSURE OF THE INVENTION

An object of the present invention is to provide gum arabic (modified gum arabic) that is modified so as to contain a large amount of dietary fiber, in particular total dietary fiber, with aiming to develop foods, drinks and medicines in which gum arabic is used as dietary fiber. Another object of the present invention to provide foods and drinks using such modified gum arabic as a dietary fiber material.

In order to distinguish gum arabic used as a material for modification from the modified gum arabic of the present invention, such gum arabic for modification is referred to as "gum arabic", "natural gum arabic" or "unmodified gum arabic" in this specification.

In order to solve the above problems, the present inventors conducted intensive research and found that the total dietary fiber content can be increased by heating natural gum arabic (*Acacia senegal* or *Acacia seyal*) under specific conditions.

The present invention has been accomplished based on that finding and encompasses the following aspects.

Item 1. Water-soluble modified gum arabic having a total dietary fiber content (measured by the AOAC method) of not less than 90%.

Item 2. Modified gum arabic according to Item 1, which has the weight-average molecular weight of not less than 1 million.

Item 3. Modified gum arabic according to Item 1 or 2, which is used as a dietary fiber material for a food, drink, or medicine.

Item 4. Modified gum arabic according to Item 1 or 2, which is used as an additive for enriching dietary fiber of a food, drink, or medicine.

Item 5. Modified gum arabic according to any one of Items 1 to 4, which is obtained by heating gum arabic.

Item 6. Modified gum arabic according to Item 5, which is obtained by heating gum arabic at 110°C for not less than 24 hours, or under conditions by which similar effects can be obtained.

Item 7. Modified gum arabic according to any one of Items 1 to 6, which is of *Acacia senegal* origin.

Item 8. A method for preparing the modified gum arabic of any one of Items 1 to 6, which comprises a step of heating gum arabic at 110°C for not less than 24 hours, or under conditions by which similar effects can be obtained.

Item 9. A dietary fiber material for a food, drink, or medicine (preferably, an oral medicine), the dietary fiber material comprising or consisting of the modified gum arabic of any one of Items 1 to 7.

Item 10. A method in which the modified gum arabic of any one of Items 1 to 7 is used as a dietary fiber material for a food, drink, or medicine (preferably, an oral medicine).

Item 11. An additive for enriching the dietary fiber in a food, drink, or medicine (preferably, an oral medicine), the additive comprising or consisting of the modified gum arabic of any one of Items 1 to 7.

Item 12. A method in which the modified gum arabic of any one of Items 1 to 7 is used as an additive for enriching the dietary fiber of a food, drink, or medicine (preferably, an oral medicine).

Item 13. A food, drink, or medicine (preferably, an oral medicine) containing the modified gum arabic of any one of Items 1 to 7 as a dietary fiber material.

Item 14. A food or drink whose dietary fiber content is increased by containing the modified gum arabic of any one of Items 1 to 7 as a dietary fiber material.

Item 15. The food, drink, or medicine (preferably, an oral medicine) according to Item 13 or 14, which is used for improving bowel movements, improving intestinal functions, improving intestinal conditions, preventing obesity, controlling blood lipid levels, reducing blood cholesterol levels, controlling blood-sugar levels or preventing the development of cancer.

Item 16. A method for increasing the dietary fiber content of a food or drink by using the modified gum arabic of any one of Items 1 to 7 as a dietary fiber material in preparing the food or drink.

Item 17. Use of the modified gum arabic of any one of Items 1 to 7 as a dietary fiber material.

Item 18. Use of the modified gum arabic of any one of Items 1 to 7 as an additive for enriching dietary fiber.

Item 19. Use of the modified gum arabic of any one of Items 1 to 7 in preparing a food, drink, or medicine (preferably, an oral medicine).

Item 20. The use of the modified gum arabic according to Item 19, wherein the food, drink, or medicine is used for improving bowel movements, improving intestinal functions, improving intestinal conditions, preventing obesity, controlling blood lipid levels, reducing blood cholesterol levels, controlling blood-sugar levels, or preventing the development of cancer.

BRIEF DESCRIPTION OF DRAWINGS

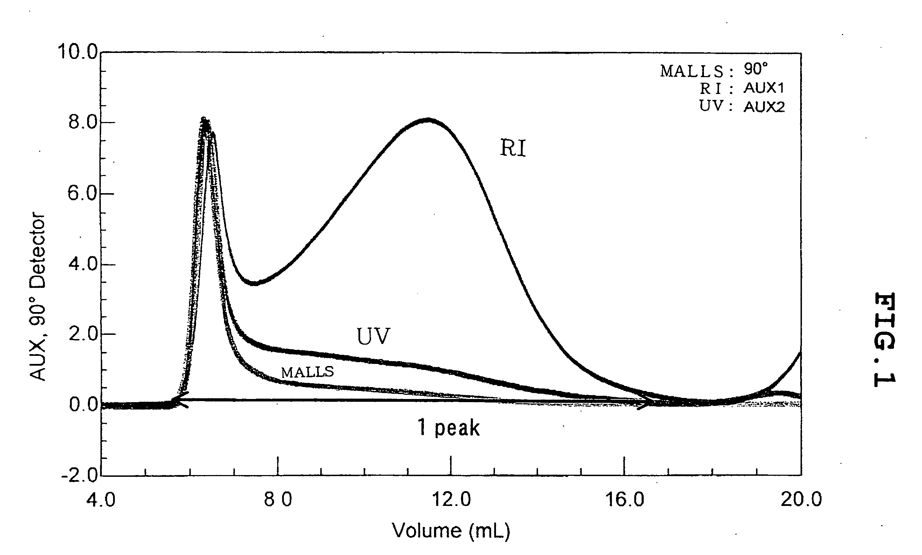

Fig. 1 shows a chromatogram obtained by subjecting modified gum arabic to GPC-MALLS (gel filtration chromatography), wherein the modified gum arabic was prepared by placing 70 kg of cracked unmodified gum arabic (*A. senegal,* particle size of 5 mm, weight-average molecular weight of $5.36 \pm 0.02 \times 10^5$) in a 100 L stainless steel drum, and heating it in an oven at 110° C for 36 hours.

BEST MODE FOR CARRYING OUT THE INVENTION

(1) Modified gum arabic

The modified gum arabic of the present invention has a total dietary fiber content (measured by the AOAC method) of not less than 90% per 100 weight % of modified gum arabic.

Total dietary fiber means indigestible polysaccharides and lignin which are not hydrolyzed by human digestive enzymes when consumed. The total dietary fiber content in the modified gum arabic of the present invention can be determined by the Prosky method (AOAC Official Method: CEREAL FOODS CHAPER 32 (2000), p.7-12, "AOAC OFFICIAL METHODS OF ANALYSIS", AOAC official method 991.43), which is a method for quantifying dietary fiber.

The Prosky method is a method for determining the quantity of total dietary fiber (including water-soluble dietary fiber and insoluble dietary fiber) in a sample. The specific method for determining the total dietary fiber content of a sample is as follows: 1 g of sample (solid) is treated stepwise with heat resistant α-amylase, protease and amyloglucosidase, and starch and proteins thereof are hydrolyzed. Water-soluble dietary fiber and insoluble dietary fiber are collectively precipitated by adding a 4 times amount of ethanol to the reaction mixture. The precipitate is filtered to collect a residue. The collected residue is washed with ethanol and acetone, dried, and the weight thereof (weight of the dried residue) is measured. The total dietary fiber content in the sample is obtained by subtracting the weight of indigestible protein and ash from the weight of the dried residue.

As a simple method, the total dietary fiber content of a sample (modified gum arabic in the case of the present invention) can be measured by using, for example, a "total dietary fiber content assay kit" (product of Biocon (Japan) Ltd.).

The modified gum arabic of the present invention has a total dietary fiber content of not less than 90 weight %, preferably not less than 91 weight %, and more preferably not less than 93 weight %. There is no upper limit thereof up to 100 weight % as long as the modified gum arabic is water-soluble as a whole. There is no limit to the percentage of insoluble dietary fiber in the total dietary fiber content as long as the modified gum arabic is water-soluble as a whole.

"Water-soluble" in this specification means that a sample can be almost completely dissolved in an excess of water, without restriction to the type of water, e.g., ion-exchanged water or ion-containing water, or to water temperature, as long as the gum arabic is soluble. Hydro-gelatinous gum arabic cannot be dissolved in water even if a large amount of water is added or by heating, and therefore the term "water-soluble" is used in the present specification to distinguish the modified gum arabic of the invention from hydro-gelatinous gum arabic, which is insoluble in water. In other words, the modified gum arabic of the invention does not include modified polymeric gum arabic that is insoluble in water, such as hydrogels, etc.

Moreover, it is preferable that the modified gum arabic of the present invention have the foresaid total dietary fiber content, be water-soluble, and be the same as or similar to unmodified gum arabic in terms of immunological reactivity. The phrase "the same as or similar to unmodified gum arabic in terms of immunological reactivity" means that the difference between the degree of immunological inhibition of the modified gum arabic and that of unmodified gum arabic is within $\pm 10\%$, as measured by indirect competitive ELISA using a quantifiable antibody (e.g., SYCC7) [Thurston, M. 1. et al., Detection of gum from *Acacia seyal* and species of combretum in mixtures with *A. senegal* using monoclonal antibodies, Food & Agric. Immunol., 10 : 237-241(1998); Thurston, M. I. et al., Effect of heat and pH on carbohydrate epitopes from *Acacia senegal* by specific monoclonal antibodies, Food & Agric. Immunol., 11 : 145-153(1999)].

The form of the modified gum arabic of the present invention is not limited and it can take any form, including blocks, beads, coarse pulverizates, granules, pellets and powders.

The modified gum arabic of the present invention can be prepared by heating gum arabic from *Acacia senegal* or *Acacia seyal* using a thermostat or a heater, such as an oven, for example, at 110°C for not less than 24 hours.

The unmodified gum arabic used as a raw material (hereunder the unmodified gum arabic may be referred to as unmodified gum arabic (*A. senegal*), unmodified gum arabic (*A. seyal*), or collectively unmodified gum arabic) is a natural resin (polysaccharide) prepared by drying a rubbery exudate obtained from the trunks and branches of *Acacia senegal* or *Acacia seyal* of the genus Acacia, family Leguminasae, or any other tree belonging to the same genus. It is also possible to use unmodified gum arabic that has been subjected to treatments, such as purification, desalting, pulverization, spray drying, etc.

Unmodified gum arabic is produced in countries of North and West Africa from Ethiopia to Senegal (Ethiopia, Sudan, Senegal, Nigeria, Niger, and Ghana), countries of East Africa such as Kenya and Uganda, the Sahara region of Africa and the basins of the tributaries of the Nile. Unmodified gum arabic produced in any of the above areas can be used in the present invention regardless of its origin.

Furthermore, unmodified gum arabic is not particularly restricted in its water content. Commercially available unmodified gum arabic undergoes a reduction in water content when dried by heating at 105°C for 6 hours (loss-on-drying) of generally not more than 40 weight %, preferably not more than 30 weight %, and more preferably not more than 20 weight %. In the present invention, unmodified gum arabic having such water contents (loss-on-drying) can be used without limitation.

Unmodified gum arabic can usually be available in the forms of blocks, beads, coarse pulverizates, granules, pellets, and powders (including spray dried powders and roller dried powders). However, in the present invention, unmodified gum arabic of any form can be used without limitation as a material to be processed. It is possible to use gum arabic powder (spray dried or roller dried) having an average particle diameter of several tens $\mu$m to several hundred $\mu$m. There is no particular upper limit to the average particle diameter but from the viewpoint of modification efficiency, the average particle diameter is preferably not more than 100 mm. The average particle diameter is preferably in the range of from 1 mm to 100 mm and more preferably from 2 mm to 50 mm.

Examples of methods for heating unmodified gum arabic include heating unmodified gum arabic at 110°C for more than 24 hours using an oven (thermostat or heater). A preferable heat treatment is such that unmodified gum arabic is heated at 110°C for not less than 48 hours. Depending on the type of the unmodified gum arabic (*A. senegal* or *A. seyal*) to be subjected to heat treatment, the upper limit to the duration of heating when heated at 110°C is usually about 72 hours. The above heating temperature and duration of heating are only examples of methods (conditions) for preparing modified gum arabic of the present invention, and therefore these conditions serve as a guideline for preparing the modified gum arabic of the present invention, and not to limit the modified gum arabic of the present invention.

In other words, as long as water-soluble modified gum arabic having the total dietary fiber content specified in the present specification can be obtained, the heating method is not limited to the above examples and heating temperature, duration of heating, heating means, and heating conditions (relative humidity, with or without being a closed system) can be selected as desired. The effects of the present invention achieved by a heat treatment conducted under the conditions described above can also be obtained by a method wherein unmodified gum arabic is heated at a temperature lower than 110°C for more than 24 hours or at a temperature higher than 110°C for a shorter time. Specifically, a method wherein the unmodified gum arabic is heated at 80°C for 3 days to 2 weeks or longer may be mentioned as one example of the former case. When the unmodified gum arabic is heated using microwave radiation instead of an oven, the same effects can be achieved in less time. In addition, a heat treatment in the absence of oxygen, such as under nitrogen displacement conditions, is advantageous because it can prevent discoloration of the gum arabic.

Furthermore, when gum arabic belonging to the *Acacia senegal* species is used as a material for the modified gum arabic of the present invention, it is preferable that such unmodified gum arabic have a weight average molecular weight of not less than 1 x 10$^6$.

The weight average molecular weight is determined by the use of gel permeation chromatography wherein three detectors, i.e., a multi angle laser light scattering (MALLS) detector, a refractive index (RI) detector and an ultraviolet (UV) detector are coupled by on-line. (In the present specification, such gel permeation chromatography is referred to as "GPC-MALLS".) According to GPC-MALLS, the molecular weight is measured by the MALLS detector, the weight of each component (composition ratio) is measured by the RI detector, and protein content is measured by the UV detector. Therefore, it is possible to obtain the molecular weight and the composition of the analyzed components without reference to a standard gum arabic of known molecular weight. For detailed principals and characteristics of GPC-MALLS, see Idris, O. H. M., Williams, P. A. Phillips, G. O. ; Food Hydrocolloids, 12, (1998) pp. 375-388.

Conditions for GPC-MALLS employed in the present invention are as below:

- Column : Superose (6HR) 10/30 (Pharmacia Biotech, Sweden)
- Flow rate : 0.5 mL/minute
- Eluant : 0.2 M NaCl
- Preparation of sample : The sample to be analyzed is diluted with the eluant (0.2 M NaCl).
- Sample concentration : 0.4% (W/V)
- Injection volume of sample solution : 100 $\mu$l
- dn/dc : 0.141
- Temperature : Room temperature
- Detector : 1. MALLS (multi angle laser light scattering) detector: DAWN DSP (manufactured by Wyatt Technology Inc., USA) 2. RI detector 3. UV detector (absorption at 214 nm)

By processing the data obtained by the GPC-MALLS conducted under the above-described conditions using software, i.e., ASTRA Version 4.5 (Wyatt Technology), parameters of the gum arabic such as the weight average molecular weight, recovery ratio (% mass), polydispersity value (P), and root mean square radius of gyration (Rg) can be obtained. When the data is processed considering all of the peaks on the chromatogram obtained using an RI detector as one peak, the obtained molecular weight is identified as the weight average molecular weight ($M_{wt}$) of the present invention (specifically, "$M_{wt}$ processed as one peak"). When the point where the RI plot begins to rise from the baseline of the chromatogram is defined as the "starting point", and the point where the RI chart falls and touches the baseline is defined as the "ending point", the aforementioned one peak of the chromatogram means the area from the starting point to the ending point.

For example, Fig. 1 shows a chromatogram obtained by subjecting modified gum arabic to GPC-MALLS (gel filtration chromatography), wherein the modified gum arabic was prepared by placing 70 kg of cracked unmodified gum arabic (*A. senegal,* particle size of 5 mm, weight-average molecular weight of 5.36 $\pm$ 0.02$\times$10$^5$) in a 100 L stainless steel drum, and heating it in an oven at 110° C for 36 hours. The horizontal axis, "Volume (mL)", indicates the cumulative volume of the eluant passing through the column and the vertical axis, 'AUX, 90° Detector', indicates the relative intensity at each detector (MALLS detector, RI detector, and UV detector). The chromatogram (MALLS chart) obtained by the MALLS detector indicates the light scattering intensity at 90°, which is dependent on the molecular weight distribution. The RI chromatogram (RI chart) obtained with the RI detector indicates the refractive index intensity, which correlates with the weight of the component(s) contained in the eluant. The chromatogram obtained by the UV detector shows the UV absorption at 214 nm, which correlates with the protein distribution.

When the data is processed considering all the peaks on the chromatogram obtained using an RI detector as one peak, the obtained molecular weight is identified as the weight average molecular weight ($M_{wt}$) of the present invention (specifically, "$M_{wt}$ processed as one peak"). When the point where the RI chart begins to rise from the baseline of the chromatogram is defined as the "starting point", and the point where the RI chart falls and touches the baseline is defined as the "ending point", the aforementioned one peak on the chromatogram means the area from the starting point to the ending point.

There is no limitation to the weight average molecular weight of the modified gum arabic of the present invention as long as it is not less than 1 million; preferably it is not less than 1.2 million, more preferably not less than 1.5 million, and still more preferably not less than 2 million. There is no specific upper limit to the weight average molecular weight as long as the modified gum arabic has total dietary fiber content of not less than 90 weight % and is soluble in water; however, it is preferably 2.5 million or less.

(2) Dietary fiber material/an additive for enriching dietary fiber

The present invention provides use of the above-descried modified gum arabic having a total dietary fiber content of not less than 90 weight % measured by the AOAC official method as a material for foods, drinks and medicines. The modified gum arabic can be used for enhancing dietary fiber (increasing the dietary fiber content) in foods, drinks, and medicines. Specifically, the present invention provides an edible additive that can be orally taken for enhancing dietary fiber (increasing the dietary fiber content) in foods, drinks, and oral medicines. In the present invention, such an edible additive is a dietary fiber material or an additive for enriching dietary fiber in foods, drinks, and medicines.

The dietary fiber material or additive for enriching dietary fiber of the present invention may consist solely of the modified gum arabic of the present invention, or comprise the modified gum arabic of the present invention as an active ingredient together with other water-soluble dietary fibers, insoluble dietary fibers, and/or food hygienically and pharmaceutically acceptable carriers or additives. In the latter case, it is preferable that the content of the modified gum arabic of the present invention be not less than 30 weight %.

Examples of water-soluble dietary fibers other than modified gum arabic are pectin, guar gum, psyllium, galactomannan, xyloglucan, locust bean gum, glucomannan, sodium alginate, chondroitin sulfate, low molecular -alginic acid, low molecular-guar gum, indigestible dextrin, polydextrose, pullulan, fiberon, etc.

Examples of insoluble dietary fibers are cellulose, wheat bran, apple fiber, sweet potato fiber, cone fiber, chitin, etc.

There is no limitation on the usable food hygienically and pharmaceutically acceptable carriers and additives, and examples thereof include dextrin, lactose, maltose, trehalose, glucose and like saccharides; sorbitol, mannitol and like sugaralcohols; and glycerol, propylene glycol and like polyhydric alcohols.

The dietary fiber material or additive for enriching dietary fiber is added with other materials as an ingredient in a process for preparing a food, drink or oral medicine to increase the dietary fiber content thereof.

(3) Foods and drinks and/or medicines

The present invention provides foods, drinks and medicines having an increased dietary fiber content by adding the modified gum arabic having a total dietary fiber content of not less than 90 weight % measured by the AOAC official method. Such foods, drinks and medicines can also be prepared by using the dietary fiber material or additive for enriching dietary fiber of the present invention instead of the above-described modified gum arabic.

There is no restriction on the types, content of modified gum arabic, and total dietary fiber content of the foods and drinks of the present invention as long as the total dietary fiber content is increased by containing the modified gum arabic of the present invention.

The content of modified gum arabic of the present invention is preferably not less than 1 weight %, more preferably not less than 5 weight %, and further more preferably not less than 10%. There is no particular upper limit of the content. However, considering the fact that the modified gum arabic of the present invention itself can be used (eaten) as health food (functional food), the upper limit thereof is 100 weight %.

The foods and drinks of the present invention are not limited and examples thereof include soft drinks, fruit drinks, milk beverages, lactic acid bacteria beverages, carbonated beverages, vegetable drinks, sports drinks, black tea beverages, green tea beverages, powder beverages, coffee beverages, cocoa beverages, soups, siruko (adzuki-bean soup with rice cake) and like beverages; puddings, jellies, yogurts and like desserts; ice creams, popsicles and like cold sweets; chewing gum, chocolate, soft candies, biscuits, cookies and like confectioneries; dressings, sauces, ketchup and like seasonings; and jams, noodles, fish cakes, syrups, breads, ready-made meals and like processed foods. The modified gum arabic of the present invention can also be provided as supplements (health foods, functional foods) in the form of tablets, capsules, pills, granules, pulvis, powders, solutions (ampuled liquid medicines).

When the foods and drinks of the present invention are provided in the form of supplements, in addition to an effective amount (the amount effective for increasing dietary fiber) of the modified gum arabic of the present invention, which serves as an active ingredient, food hygienically acceptable carriers or other additives may be added.

The amount of modified gum arabic contained in the foods or drinks and the dosage thereof are not limited and can be suitably selected depending on the type of the food or drink, intended effects and the degree thereof, and other conditions. The dosage varies depends on the type of the food or drink, but a preferable dosage is about 1 to 100 g per day for an adult with a body weight of 60 kg as calculated by the amount of modified gum arabic.

By containing a large amount of dietary fiber, the foods and drinks of the present invention have various physiological functions or disease prevention effects such as reducing blood cholesterol levels, controlling blood lipid levels, improving bowel movements (including relieving constipation), improving intestinal functions by improving the intestinal conditions, preventing obesity, controlling blood-sugar levels, preventing the development of cancer, etc. Furthermore, since the modified gum arabic has a low viscosity, it does not adversary affect the texture of the foods or drinks of the present invention even when a large amount of the modified gum arabic is included, and it does not cause any side effects such as diarrhea when a large amount thereof is consumed.

By containing the modified gum arabic of the present invention, the medicines encompassed in the present invention have physiological functions or disease prevention effects such as reducing blood cholesterol levels, controlling blood lipid levels, improving bowel movements (including alleviating constipation), improving intestinal functions by improving the intestinal conditions, preventing obesity, controlling blood-sugar levels, preventing the development of cancer, etc. There is no limitation on the content of the modified gum arabic and the total dietary fiber content as long as the medicines of the present invention have such a function or effect. The medicines encompassed in the present invention are oral medicines. Such medicines can be mixed, formed, or prepared as tablets, pills, pulvis, powders, granules, capsules, dry syrups or the like solid solids; or as solutions, suspensions, emulsions, syrup or the like liquids.

A medicine of the present invention may contain pharmacologically acceptable carriers or additives together with modified gum arabic. Examples of carriers used in preparing such medicines include excipients, diluting agents, binders, humectants, disintegrators, disintegration inhibitors, absorption accelerators, lubricants, solubilizers, buffers, emulsifiers, and suspensions, i.e., carriers typically used depending on the dosage form of the preparation. Examples of usable additives include stabilizers, preservatives, buffers, isotonicity agents, chelating agents, pH adjustors, surfactants, coloring agents, perfumes, flavors, sweetening agents, etc., i.e., additives typically used depending on the dosage form of the preparation.

The amount of modified gum arabic contained in the medicine of the present invention varies depending on the form of the medicine and the administration route thereof and cannot be generally be specified, but is preferably not less than 5 weight % of the final pharmaceutical preparation, and more preferably not less than 10 weight %. There is no upper limit of the content thereof, but in the light of the fact that the modified gum arabic of the present invention itself can be used as a medicine, the upper limit is 100 weight %.

The dosage of the medicine of the present invention is not limited and can be suitably selected depending on the intended effects, duration of administration, administration method, duration of treatment, patient's age and sex, and other conditions. The dosage varies depending on the administration route, but can be suitably selected from the range about 1 to 100 g per day for an adult with a body weight of 60 kg as calculated by the amount of a pharmaceutically effective ingredient.

Examples

The details of the present invention are explained below with reference to Examples. However, the present invention is not limited to or by these Examples. In the Examples, unless otherwise specified, "parts" means "parts by weight" and "%" means "weight %".

Experimental Example 1

(1) Preparation of modified gum arabic

One kg of cracked gum arabic belonging to the *Acacia senegal* species (unmodified gum arabic from *A. senegal*: 'Sample 1', particle size of 5 mm) was placed in an unsealed stainless steel container, and heated at 110°C for 24 hours or 48 hours using an oven (gum arabic samples heated for 24 hours and 48 hours are referred to as 'Sample 1/24' and 'Sample 1/48', respectively).

(2) Content of dietary fiber

The total dietary fiber contents of the above-obtained gum arabic samples (Sample 1, Sample 1/24, Sample 1/48) were measured by the Prosky (AOAC) method.

Specifically, total dietary fiber contents of two specimens per sample each containing 1 g gum were measured according to the following procedure. Note that one of the two specimens was for measuring the content of indigestible proteins and the other was for measuring the ash content:

1. Two specimens each containing 1.0 g were weighed to 0.1 mg precision and each specimen was placed in a 400 mL beaker.

2. To 1.0 g of each specimen were added 50 mL of 0.08 M phosphate buffer (pH 6.0) and 0.1 mL of heat resistant a amylase (manufactured by Novo, termamyl 120L). The top of the beaker was covered with aluminum foil, the beaker was shaken every 5 minutes in a boiling-water bath, and the mixture was reacted for 30 minutes after the temperature of the content of the beaker reached 95°C.

3. The temperature of the content of the beaker was reduced to room temperature, 10 mL of 0.275 N sodium hydroxide solution was added, and the mixture was adjusted to pH 7.5 ± 0.2. A protease solution (0.1 mL, a solution obtained by dissolving protease (manufactured by Sigma, product name of P3910) in a 0.08 M phosphate buffer (pH6.0) so that the content thereof was 50 mg/mL) was added to the mixture, the top of the beaker was covered with aluminum foil, the beaker was shaken in a water bath at 60°C, and the mixture was reacted for 30 minutes after the temperature at the center of the mixture reached 60°C.

4. When the temperature of the mixture was reduced to room temperature, 10 mL of 0.325 M aqueous hydrochloric acid was added, and the mixture was adjusted to pH 4.0 to 4.6. To the mixture was added 0.3 mL of amyloglucosidase solution (amyloglucosidase, manufactured by Sigma, product name of P-9913), the top of the beaker was covered with aluminum foil, the beaker was shaken in a water bath at 60°C, and the mixture was reacted for 30 minutes after the temperature at the center of the mixture reached 60°C.

5. 280 mL of 95% aqueous ethanol that had been measured and heated to 60°C in advance was added to the enzyme reaction solution in the beaker, and the beaker was allowed to stand under room temperature for exactly 60 minutes, obtaining a precipitate.

6. Using a wash bottle, 78 v/v% aqueous ethanol was poured into a glass filter accommodating celite (celite 545, manufactured by Sigma, C-8656) to suspend the celite. The suspension was subjected to vacuum suction to form a uniform mat-shaped filter layer. The enzyme reaction solution containing the precipitate generated by ethanol was poured onto the filter and subjected to suction filtration. The residue was sequentially washed with 20 mL of 78 v/v% aqueous ethanol three times, with 10 mL of 95 v/v% aqueous ethanol twice, and with 10 mL of acetone twice.

7. The filter containing the residue was dried over night in a vacuum oven (70°C), cooled in a desiccator, weighed to 0.1 mg precision, and the actual amount of the residue was obtained by subtracting the weight of the filter and celite therefrom.

8. The residue of one of the two specimens was scraped off together with celite. The nitrogen content of the residue was determined by a semimicro-Kjeldahl method, and the protein content thereof was obtained by multiplying by a conversion factor of 6.25. The other residue was subjected to ashing at 525°C for 5 hours, cooled in a desiccator, weighed to 0.1 mg precision, and the ash content in the residue was obtained.

9. The blank residue, blank protein, and blank ash weights were obtained by conducting steps 2 to 8 in the absence of a sample.

The total dietary fiber content % was calculated using the obtained weights by the following formulae.

$$\text{Total dietary fiber content \%} = \frac{\text{Residue mg} - \left\{ \left[ \dfrac{\text{Protein residue\% + Ash residue\%}}{100} \right] \times \text{Residue mg} \right\} - \text{Blank}}{\text{Sample mg}} \times 100$$

$$\text{Protein residue\%} = \left[ \frac{\text{Protein residue mg}}{\text{Residue mg}} \right] \times 100$$

$$\text{Ash residue\%} = \left[ \frac{\text{Ash residue mg}}{\text{Residue mg}} \right] \times 100$$

$$\text{Blank} = \text{Blank residue mg} - \left\{ \left[ \frac{\text{Blank protein\% + Blank ash\%}}{100} \right] \times \text{Blank residue mg} \right\}$$

$$\text{Blank protein\%} = \left[ \frac{\text{Blank protein mg}}{\text{Blank residue mg}} \right] \times 100$$

$$\text{Blank ash\%} = \left[ \frac{\text{Blank ash mg}}{\text{Blank residue mg}} \right] \times 100$$

(3) Measurement of weight-average molecular weight

The gum arabic samples (Sample 1, Sample 1/24, Sample 1/48) were subjected to gel filtration chromatography employing the GPC-MALLS (Multi Angle Laser Light Scattering) method under the below specified conditions, obtaining chromatograms.

- Column : Superose (6HR) 10/30 (Pharmacia Biotech)
- Flow rate : 0.5 mL/minute
- Eluant : 0.2 M NaCl
- Preparation of sample : The sample to be analyzed was diluted with the eluant (0.2 M NaCl).
- Sample concentration : 0.4% (W/V)
- Injection volume of sample solution : 100 μl
- dn/dc : 0.141
- Temperature : Room temperature
- Detector : 1. MALLS (multi angle laser light scattering) detector: DAWN DSP (manufactured by Wyatt Technology Inc.) 2. RI detector 3. UV detector

(absorption at 214 nm)

By processing the data obtained by the GPC-MALLS conducted under the above-described conditions using software, i.e., ASTRA Version 4.5 (Wyatt Technology), weight-average molecular weight ($M_{wt}$ processed as one peak) was obtained. When the data is processed considering the all the peaks on the chromatogram obtained using an RI detector as one peak, the obtained molecular weight is identified as the weight average molecular weight ($M_{wt}$) of the present invention (specifically, "$M_{wt}$ processed as one peak"). When the point where the RI plot begins to rise from the baseline of the chromatogram is defined as the "starting point", and the point where the RI chart falls and touches the baseline is defined as the "ending point", the aforementioned one peak of the chromatogram means the area from the starting point to the ending point.

Table 1 shows the total dietary fiber contents and weight-average molecular weights ($M_{wt}$ processed as one peak) of the gum arabic samples (Sample 1, Sample 1/24, and Sample 1/48).

Table 1

| Sample | Weight-average molecular weight | Increase in weight-average molecular weight (%) | Total dietary fiber content (AOAC)% | Increase in total dietary fiber content(%) |
|---|---|---|---|---|
| Sample 1 | $5.15 \pm 0.18 \times 10^5$ | 100 | 84.0 | 100 |
| Sample 1/24 | $1.15 \pm 0.21 \times 10^6$ | 223.3 | 90.0 | 107.1 |
| Sample 1/48 | $1.91 \text{t} 0.17 \times 10^6$ | 370.8 | 91.0 | 108.3 |

As is clear from the results, the total dietary fiber contents in the modified gum arabic of the present invention of Samples 1/24 and 1/48 increased by 7.1 weight %(107.1%-100%=7.1%) and 8.3 weight % (108.3%-100% =7.1%) respectively from the total dietary fiber content (100 weight %) of the unmodified gum arabic (Sample 1). Furthermore, the weight-average molecular weights ($M_{wt}$ processed as one peak) were increased by 123.3% (223.3%-100% =123.3%) and 270.8% (370.8%-100%=270.8%) respectively.

Experimental Example 2 Immunoreaction of modified gum arabic

Immunoreactivities of the gum arabic samples derived from *A. senegal* (Sample 1, Sample 1/24, and Sample 1/48) obtained in Experimental Example 1 were evaluated. More specifically, immunoreactivities of the gum arabic samples were measured using plates immobilized with one of each kind of gum arabic sample (concentrations: 0.005 mg/mL, 0.01 mg/mL, 0.05 mg/mL, 0.1 mg/mL, 0.5 mg/mL, 1 mg/mL, and 5 mg/mL) by indirect competitive ELISA according to Thurston, M.I. et al. [Thurston, M.I., et al.; Detection of gum from *Acacia seyal* and species of combretum in mixtures with *A. senegal* using monoclonal antibodies, Food & Agric. Immunol., 10:237-241 (1998); Thurston, M.I., et al., Effect of heat and pH on carbohydrate epitopes from *Acacia senegal* by specific monoclonal antibodies, Food & Agric. Immunol., 11:145-153 (1999)].

Before conducting the ELISA, monoclonal antibodies having no specificity and showing quantitative cross-reactivity to any species of gum arabic were prepared. More specifically, an adjuvant was added to a saline solution containing 1 mg/mL of gum arabic from *Acacia senegal* to prepare an immunogen. Balb/c mice were given intraperitoneal injections of the immunogen three times at 2-week intervals. The splenocytes of the mice with highly raised antibody titers were extracted and fused with myeloma cells in polyethylene glycol. After incubating the obtained cells on an incubation plate for 10 days, the hybridoma cells were selected based on specificity to antibodies produced in the supernatant of the proliferated hybridoma cells. The selected hybridoma cells were incubated for another 10 days on the incubation plate and specific hybridoma cells were selected by the same procedure. Those hybridoma cells producing only antibodies having the above specificity (such antibodies were named SYCC7) were finally selected.

The ELISA was conducted using the thus-prepared SYCC7 antibody by the following steps.

1. 1 mg/mL and 5 mg/mL of solutions of the three samples (Sample 1, Sample 1/24, and Sample 1/48) were diluted to 10-fold, 100-fold and 1000-fold.
2. 200 $\mu$l each of the above-obtained solutions was added into wells of a plastic plate, and immobilized at 4°C for 1 hour. The wells were washed with saline solution, blocked with 0.3% casein-containing saline solution and washed with 0.05% Tween 20-containing saline solution.
3. Culture supernatant of the prepared hybridoma cells was added and immobiized for 1 hour. After washing as per the above-mentioned procedure, the wells were subsequently immobilized with peroxidase-labeled goat anti-mouse antibodies (SIGMA, diluted 1,000-fold with saline solution) for 1 hour.
4. After washing, tetramethylbenzidine was added as a substrate to the wells and the color intensity was measured as UV absorption at 450 nm ($UV_{450}$nm).

The inhibition ratio (%) for each sample at various concentrations was determined by comparison with the UV absorption at 450nm ($UV_{450nm}$) of natural gum arabic (unmodified gum arabic: Sample 1) from *A. seyal*, which is defined as 100% inhibition.

Test results show that the modified gum arabic of the present invention (Sample 1/24 and Sample 1/48) and natural gum arabic (Sample 1) have the same or similar immunological reactivities since differences in the immunological inhibition ratio therebetween were within ±10% over the tested concentration range, and therefore negligible.

Experimental Example 3

Using gum arabic (sample 1) and modified gum arabic (sample 1/24 and sample 1/48), aqueous solutions of various concentrations were prepared and the viscosities thereof were measured. Specifically, 10 g of a gum arabic sample (sample 1, sample 1/24, sample 1/48) was dissolved in 90 g of water, giving a 10 weight % gum arabic aqueous solution. In the same manner, 20 weight % and 30 weight % gum arabic aqueous solutions were prepared. Each aqueous solution was placed in a 100 mL screw cap vial, and the viscosities (mPa · S) were measured using a B-type rotational viscometer (BM type, manufactured by TOKIMEC INC., rotor No. 1 with 60 rpm), at 20°C. Table 2 shows the results.

Table 2

| Sample | viscosity (mPa·S) | | |
|---|---|---|---|
| | 10% aqueous solution | 20% aqueous solution | 30% aqueous solution |
| Sample 1 | 11 | 40 | 192 |
| Sample 1/24 | 12 | 45 | 240 |

Table continued

| Sample | viscosity (mPa·S) | | |
|---|---|---|---|
| | 10% aqueous solution | 20% aqueous solution | 30% aqueous solution |
| Sample 1/48 | 13 | 57 | 300 |

Note that, although this depends somewhat on the products, a viscosity of 300 mPa·S is typical of gum syrups and corn soups, and is a relatively low viscosity.

As is clear from the results, the viscosities of the sample of the modified gum arabic of the present invention (Sample 1/24 and Sample 1/48) were higher than that of the unmodified gum arabic (Sample 1). This increase in the viscosity affects the transfer of foodstuffs from the stomach to small intestine. When absorption of various nutrients and food compositions is to be controlled, for example, the speed of absorbing glucose from the small intestine is reduced and the blood-sugar level rise is slowed down. It is believed that an insulin-sparing action is thus achieved. Having a high viscosity is believed to affect the villus of the small intestine and to accelerate the metabolism therein. Furthermore, it can prevent gastric ulcer by protecting the stomach walls.

Example 1 Preparing gum arabic powder

The modified gum arabic obtained in Experimental Example 1 can be prepared into a gum arabic powder by the following steps. Various kinds of foods and drinks as shown in Examples 2 to 4 can be prepared by using such a gum arabic powder. <Modified gum arabic powder>

Modified gum arabic (Sample 1/24, 1000 g) was dissolved in 1500 g of water, giving a gum arabic aqueous solution. The aqueous solution was spray dried using a spray drier (product of ANHYDRO, inlet at 140°C and outlet at 80°C), preparing 950 g of modified gum arabic powder.

Example 2 Fruit juice containing soft drinks (Brix 10°)

After mixing the following ingredients, the mixture was packed in bottles and sterilized at 85°C for 30 minutes, preparing a soft drink that contained fruit juice.

<Formula>

| | |
|---|---|
| High fructose corn syrup (Brix 75°) | 13.3 (weight %) |
| Citric acid | 0.1 |
| Sodium citrate | 0.05 |
| Vitamin C | 0.05 |
| 5-fold concentrated citrus fruits-containing transparent juice | 6.0 |
| Modified gum arabic powder (Example 1) | 3.0 |
| Water | 77.5 |
| Total | 100.0 (weight %) |

Example 3 Hard candies

<Formula>

| | |
|---|---|
| Sugar | 59.0 (weight %) |
| Glucose syrup | 40.0 |
| Water | 20.0 |
| Modified gum arabic powder (Example 1) | 1.0 |

Table continued

| | |
|---|---|
| Citric acid | 0.4 |
| L-ascorbic acid | 0.01 |
| Pineapple flavor | 0.15 |
| Total (after preparation) | 100.0 (weight %) |

Sugar, glucose syrup and water were mixed, melted by heating to 155°C, and cooled to 125°C. Modified gum arabic powder, citric acid, L-ascorbic acid and pineapple flavor were sequentially added to the mixture, and formed into a desired shape, giving hard candies.

Example 4 Supplement(pharmaceutical preparation containing dietary fiber)

The following powdered ingredients were mixed, and supplements (tablets) were prepared using a tableting machine (tabletting pressure of 1 t).

<Formula>

| | |
|---|---|
| Modified gum arabic powder (Example 1) | 30 (weight %) |
| Sorbitol | 69 |
| Sucrose esters of fatty acids | 1 |
| Total | 100 (weight %) |

INDUSTRIAL APPLICABILITY

The modified gum arabic of the present invention is modified so as to contain dietary fiber at a high content, such as a total dietary fiber content of not less than 90 weight %. In recent years, many researchers have indicated that an insufficient dietary fiber intake is relevant to the increase in patients suffering from diabetes, obesity, arteriosclerosis and the like lifestyle-induced diseases. Therefore, the physiological functions of dietary fiber have been attracting attention, and the necessity of intaking dietary fiber has been highlighted. The modified gum arabic of the present invention can increase the dietary fiber content of foods or drinks by being added to foods or drinks as a dietary fiber material or as an additive for enriching dietary fiber. This allows foods and drinks to have various physiological functions (e.g., reducing blood cholesterol levels, improving bowel movements, improving intestinal conditions, improving intestinal functions, controlling blood lipid levels, and controlling blood-sugar levels) or an effect of preventing the development of cancer. Furthermore, because of its physiological functions (e.g., reducing blood cholesterol levels, improving bowel movements, improving intestinal conditions, improving intestinal functions, controlling blood lipid levels, and controlling blood-sugar levels) or an effect of preventing the development of cancer, the modified gum arabic of the present invention can be used as a material for medicine having such effects. Furthermore, the modified gum arabic of the present invention has a low viscosity even at high concentration. Therefore, the modified gum arabic of the present invention can be added to foods or drinks without adversely affecting their texture, making it possible to take a large amount of dietary fiber.

The present invention provides foods and drinks having an increased dietary fiber content by containing modified gum arabic terein. By containing a large amount of dietary fiber, the foods and drinks of the present invention can be provided as health foods or functional foods having various physiological functions (e.g., reducing blood cholesterol levels, improving bowel movements, improving intestinal conditions, improving intestinal functions, controlling blood lipid levels, and controlling blood-sugar levels) or an effect of preventing the development of cancer due to the dietary fiber contained therein. Furthermore, the present invention can provide medicines having various physiological functions (e.g., reducing blood cholesterol levels, improving bowel movements, improving intestinal conditions, improving intestinal functions, controlling blood lipid levels, and controlling blood-sugar levels) or an effect of preventing the development of cancer due to the dietary fiber contained therein.

**Claims**

1. Water-soluble modified gum arabic having a total dietary fiber content measured by the AOAC method of not less than 90%.

2. Modified gum arabic according to Claim 1, which has the weight-average molecular weight of not less than 1 million.

3. Modified gum arabic according to Claim 1, which is used as a dietary fiber material for a food, drink, or medicine.

4. Modified gum arabic according to Claim 1, which is used as an additive for enriching dietary fiber of a food, drink, or medicine.

5. Modified gum arabic according to Claim 1, which is obtained by heating unmodified gum arabic.

6. Modified gum arabic according to Claim 5, which is obtained by heating unmodified gum arabic at 110°C for not less than 24 hours, or under conditions by which similar effects can be obtained.

7. Modified gum arabic according to Claim 1, which is of *Acacia senegal* origin.

8. A method for preparing the modified gum arabic of Claim 1, which comprises a step of heating unmodified gum arabic at 110°C for not less than 24 hours, or under conditions by which similar effects can be obtained.

9. A dietary fiber material for a food, drink, or medicine, the dietary fiber material comprising or consisting of the modified gum arabic of Claim 1.

10. A method in which the modified gum arabic of Claim 1 is used as a dietary fiber material for a food, drink, or medicine.

11. An additive for enriching the dietary fiber for a food, drink, or medicine, the additive comprising or consisting of the modified gum arabic of Claim 1.

12. A method in which the modified gum arabic of Claim 1 is used as an additive for enriching the dietary fiber of a food, drink, or medicine.

13. A food, drink, or medicine containing the modified gum arabic of Claim 1 as a dietary fiber material.

14. A food or drink whose dietary fiber content is increased by containing the modified gum arabic of Claim 1 as a dietary fiber material.

15. The food, drink, or medicine according to Claim 13, which is used for improving bowel movements, improving intestinal functions, improving intestinal conditions, preventing obesity, controlling blood lipid levels, reducing blood cholesterol levels, controlling blood-sugar levels or preventing the development of cancer.

16. A method for increasing the dietary fiber content of a food or drink by adding the modified gum arabic of Claim 1 as a dietary fiber material in preparing the food or drink.

17. Use of the modified gum arabic of Claim 1 as a dietary fiber material.

18. Use of the modified gum arabic of Claim 1 as an additive for enriching dietary fiber.

19. Use of the modified gum arabic of Claim 1 in preparing a food, drink, or medicine.

20. Use of the modified gum arabic according to Claim 19, wherein the food, drink, or medicine is used for improving bowel movements, improving intestinal functions, improving intestinal conditions, preventing obesity, controlling blood lipid levels, reducing blood cholesterol levels, controlling blood-sugar levels, or preventing the development of cancer.

# FIG.1

MALLS : 90°
RI : AUX1
UV : AUX2

RI

UV

MALLS

1 peak

Volume (mL)

AUX, 90° Detector

**EP 1 612 225 A1**

<table>
<tr><td colspan="2" align="center">INTERNATIONAL SEARCH REPORT</td><td>International application No.<br>PCT/JP2004/005146</td></tr>
</table>

A. CLASSIFICATION OF SUBJECT MATTER
Int.Cl⁷ C08B37/00, A61K31/736, A61P1/14, 35/00, 3/06, 3/10, A23L1/308

According to International Patent Classification (IPC) or to both national classification and IPC

B. FIELDS SEARCHED

Minimum documentation searched (classification system followed by classification symbols)
Int.Cl⁷ C08B37/00, A61K31/736, A61P1/14, 35/00, 3/06, 3/10, A23L1/308

Documentation searched other than minimum documentation to the extent that such documents are included in the fields searched

Electronic data base consulted during the international search (name of data base and, where practicable, search terms used)
CA(STN), JICST(JOIS)

C. DOCUMENTS CONSIDERED TO BE RELEVANT

| Category* | Citation of document, with indication, where appropriate, of the relevant passages | Relevant to claim No. |
|---|---|---|
| A | JP 2000-166489 A (The Nisshin Oil Mills, Ltd.), 20 June, 2000 (20.06.00), Claim 1 (Family: none) | 1-20 |
| A | JP 2-49001 A (Kabushiki Kaisha Shinshu Kagaku Kogyosho), 19 February, 1990 (19.02.90), Claims 1 to 2 (Family: none) | 1-20 |
| A | AKEO et al., "Arabia Gum no Seiri Kino", Food chemicals, 2002-6, 2002, page 85, left column, line 29 | 1-20 |

☒ Further documents are listed in the continuation of Box C.　　☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>09 July, 2004 (09.07.04) | Date of mailing of the international search report<br>27 July, 2004 (27.07.04) |
|---|---|
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

International application No.

PCT/JP2004/005146

C (Continuation). DOCUMENTS CONSIDERED TO BE RELEVANT

| Category* | Citation of document, with indication, where appropriate, of the relevant passages | Relevant to claim No. |
|---|---|---|
| A | Choroid Naturals International, "Fiber Gum -Tennen Yurai de Kohan'i ni Riyo Kano na Iyosei Syokumotsu Sen'i-", Food chemicals, 1997-7, 1997, page 103, right column, line 3 | 1-20 |

Form PCT/ISA/210 (continuation of second sheet) (January 2004)